# EUROPEAN PATENT APPLICATION

(11) **EP 2 383 693 A1**
(43) Date of publication of application: **02.11.2011**
(21) Application number: 10731242.3
(22) Date of filing: 13.01.2010
(51) Int. Cl.: G06Q 50/00, G06F 3/048

(54) **PROJECT INFORMATION DISPLAY, PROJECT INFORMATION DISPLAY PROGRAM, AND ELECTRONIC MEDICAL RECORD INFORMATION DISPLAY**

(30) Priority: 13.01.2009 JP 2009004511
(71) Applicant: Wise Solutions Inc., Sendai-shi, Miyagi 980-0801 (JP); Kondo, Yoshiaki, Sendai-shi, Miyagi 989-3204 (JP)
(72) Inventor: KONDO, Yoshiaki, Sendai-shi Miyagi 989-3204 (JP); TATE, Masatoshi, Sendai-shi Miyagi 981-3214 (JP)
(74) Representative: HOFFMANN EITLE
(86) International application number: PCT/JP2010/050251
(87) International publication number: WO 2010/082573

(57) **Abstract**

Provided are a project information display device, a project information display program, and an electronic medical record information display device which are capable of displaying project information for project management while allowing time spans to be changed by a simple operation to time spans a user desires, and which include: a time scale display information generation unit (31) configured to generate time scale display information including a time scale display area and a time span controller area for performing an operation to lengthen or shorten a time span of a time scale; a display information update unit (40) configured to, when the operation to lengthen or shorten the time span of the time scale is performed in the time span controller area, update the time scale display information such that the time span of the time scale is lengthened or shortened based on the operation; and a display screen information generation unit (33) configured to generate display information from the generated or updated time scale display information and electronic medical record information corresponding to time information of the time scale display information.

## Description

### TECHNICAL FIELD

The present invention relates to a project information display device, a project information display program, and an electronic medical record information display device for displaying management information on projects in various fields.

### BACKGROUND ART

Heretofore, applications for managing projects have been developed and utilized in various fields.

One example of the project management applications is an electronic medical record that manages a patient's treatment plan in medical practice.

By utilizing this electronic medical record, one can save a data storage space and quickly browse past information on a patient of interest on a display screen, as compared to utilizing a paper medical record.

To quickly browse information on a patient, a tool called clinical pathway can be utilized as described in Patent Document 1. This clinical pathway is a tool that organizes medical information in a schedule table and makes it easier to browse the course and plan of treatment.

Moreover, by using a technique described in Patent Document 2 or Patent Document 3, schedules of various kinds of information for a patient can be known at a glance. Further, the technique allows the information to be shared with other medical staffs involved in the treatment, and also allows the medical staffs to input and change data when necessary.

### PRIOR ART DOCUMENTS

### PATENT DOCUMENTS

Patent Document 1: Japanese Patent Application Publication No. 2003-108661
Patent Document 2: Japanese Patent No. 2706645
Patent Document 3: Japanese Patent No. 2815346 Patent Document 4: Japanese Patent Application Publication No. Hei 11-212700

### SUMMARY OF THE INVENTION

### PROBLEMS TO BE SOLVED BY THE INVENTION

However, the techniques described in Patent Documents 1 to 3 listed above are ones in which various kinds of information are displayed within cells in a schedule table fixed in day or hour time units. Thus, it is impossible to handle a case where one desires to display information in a. shorter time unit, e.g., in minutes or seconds, or an opposite case where one desires to display long-term information in years.

In a case of, for example, an electronic medical record managing a treatment plan in medical care, each action constituting the treatment plan may be related dependently and exclusively to a different action on a short unit time basis. In order to clearly show these kinds of information, it may be necessary to display them in minutes or seconds.

In an invention described in Patent Document 4, information can be displayed in a time scale of a desired display mode, such as a year, month-, or day-based display mode, by changing the display mode through manipulation of GUI widgets displayed on a display screen. Still, there is a need for a technique that makes it even easier to change the time spans displayed in the time scale of any of these display modes.

Thus, an object of the present invention is to provide a project information display device, a project information display program, and an electronic medical record information display device which are capable of displaying project information for project management while allowing the time spans to be changed by a simple operation to time spans a user desires.

### MEANS FOR SOLVING THE PROBLEMS

In order to achieve the above object, a project information display device of the present invention is characterized by comprising: a storage unit configured to store calendar information, and project information, the calendar information containing date information, the project information including execution time information indicating a time at which an action is executed in a project; a time scale display information generation unit configured to acquire, from the storage unit, the calendar information on a period specified as a display period and to generate time scale display information including a time scale display area for displaying a time scale, and a time span controller area being a display area for performing an operation to lengthen or shorten a time span of the time scale; a display information update unit configured to update the time scale display information upon detecting the operation to lengthen or shorten the time span of the time scale in the time span controller area of the time scale display information generated and displayed by the time scale display information generation unit, the time scale display information being updated such that the time span of the time scale in the time scale display area is lengthened or shortened based on the operation; a project display information generation unit configured to acquire project information from the storage unit and to generate project display information when the time scale display information is generated by the time scale display information generation unit, and to update project display information when the time scale display information is updated by the display information update unit, the project information corresponding to the time scale in the time scale display information and containing the execution time information in the display period, the project display information allowing the project information to be displayed corresponding to the time span of the time scale, the project display information being updated such that the project information is displayed correspondingly to the time span of the updated time scale; a display information generation unit configured to generate display screen information including any one of the time scale display information generated by the time scale display information generation unit and the time scale display information updated in the display information update unit, and any one of the project display information generated by the project display information generation unit and the project display information updated by the project display information generation unit; and a display unit configured to display the display screen information generated by the display information generation unit.

Moreover, a project information display program of the present invention causes a computer to execute: a storage function configured to store calendar information, and project information, the calendar information containing date information, the project information containing execution time information indicating a time at which an action is executed in a project; a time scale display information generation function configured to acquire, from information stored by the storage function, the calendar information on a period specified as a display period and to generate time scale display information including a time scale display area and a time span controller area, the time scale display area being where a time scale is displayed, the time span controller area being a display area for performing an operation to lengthen or shorten a time span of the time scale; a display information update function configured to update the time scale display information upon detecting the operation to lengthen or shorten the time span of the time scale, in the time span controller area of the time scale display information generated and displayed by the time scale display information generation function, the time scale display information being updated such that the time span of the time scale in the time scale display area is lengthened or shortened based on the operation; a project display information generation function configured to acquire project information from information stored by the storage function and to generate project display information when the time scale display information is generated by the time scale display information generation function, and to update project display information when the time scale display information is updated by the display information update function, the project information corresponding to the time scale in the time scale display information and containing the execution time information in the display period, the project display information allowing the project information to be displayed corresponding to the time span of the time scale, the project display information being updated such that the project information is displayed correspondingly to the time span of the updated time scale; a display information generation function configured to generate display screen information including any one of the time scale display information generated by the time scale display information generation function and the time scale display information updated in the display information update function, and any one of the project display information generated by the project display information generation function and the project display information updated by the project display information generation function; and a display function configured to display the display screen information generated by the display information generation function.

Furthermore, an electronic medical record information display device of the present invention is characterized by comprising: a storage unit configured to store calendar information, and electronic medical record information, the calendar information containing date information, the electronic medical record information containing execution time information indicating a time at which a medical action is executed; a time scale display information generation unit configured to acquire, from the storage unit, the calendar information on a period specified as a display period and to generate time scale display information including a time scale display area and a time span controller area, the time scale display area being where a time scale is displayed, the time span controller area being a display area for performing an operation to lengthen or shorten a time span of the time scale; a display information update unit configured to update the time scale display information upon detecting the operation to lengthen or shorten the time span of the time scale in the time span controller area of the time scale display information generated and displayed by the time scale display information generation unit, the time scale display information being updated such that the time span of the time scale in the time scale display area is lengthened or shortened based on the operation; an electronic medical record display information generation unit configured to acquire electronic medical record information from the storage unit and to generate electronic medical record display information when the time scale display information is generated by the time scale display information generation unit, and to update project display information when the time scale display information is updated by the display information update unit, the electronic medical record information corresponding to the time scale in the time scale display information and containing the execution time information in the display period, the electronic medical record display information allowing the electronic medical record information to be displayed corresponding to the time span of the time scale, the electronic medical record display information being updated such that the electronic medical record information is displayed correspondingly to the time span of the updated time scale; a display information generation unit configured to generate display screen information including any one of the time scale display information generated by the time scale display information generation unit and the time scale display information updated in the display information update unit, and any one of the electronic medical record display information generated by the electronic medical record display information generation unit and the electronic medical record display information updated by the electronic medical record display information generation unit; and a display unit configured to display the display screen information generated by the display information generation unit.

### MEANS FOR SOLVING THE PROBLEMS

Accordingly, with the project information display device, the project information display program, and the electronic medical record information display device of the present invention, it is possible to display project information for project management while allowing the time spans to be changed by a simple operation to time spans a user desires.

### BRIEF DESCRIPTION OF THE DRAWINGS

[Fig. 1] Fig. 1 is a block diagram showing the configuration of an electronic medical record display device as a project information display device in one embodiment of the present invention.
[Fig. 2] Fig. 2 is an explanatory diagram showing the structure of each COA utilized in the electronic medical record display device as the project management display device in the embodiment of the present invention.
[Fig. 3] Fig. 3 is an explanatory diagram showing the positions of information pieces in a COA on a time line displayed by the electronic medical record display device as the project management display device in the embodiment of the present invention.
[Fig. 4] Fig. 4 is a flowchart showing operations of the electronic medical record display device as the project management display device in the embodiment of the present invention.
[Fig. 5] Fig. 5 is a diagram showing time scale display information displayed by the electronic medical record display device as the project management display device in the embodiment of the present invention.
[Fig. 6] Fig. 6 is a diagram showing time scale display information and electronic medical record information displayed by the electronic medical record display device as the project management display device in the embodiment of the present invention.
[Fig. 7] Part (a) of Fig. 7 is a diagram showing the time scale display information and electronic medical record information displayed in the electronic medical record display device as the project management display device in the embodiment of the present invention, and Part (b) of Fig. 7 is a diagram showing the time scale display information and the electronic medical record information updated in the electronic medical record display device.
[Fig. 8] Part (a) of Fig. 8 is a diagram showing the time scale display information and electronic medical record information displayed in the electronic medical record display device as the project management display device in the embodiment of the present invention, and Part (b) of Fig. 8 is a diagram showing the time scale display information and the electronic medical record information updated in the electronic medical record display device.
[Fig. 9] Part (a) of Fig. 9 is a diagram showing the time scale display information and electronic medical record information displayed in the electronic medical record display device as the project management display device in the embodiment of the present invention, and Part (b) of Fig. 9 is a diagram showing the time scale display information and the electronic medical record information updated in the electronic medical record display device.
[Fig. 10] Part (a) of Fig. 10 is a diagram showing the time scale display information and electronic medical record information displayed in the electronic medical record display device as the project management display device in the embodiment of the present invention, and Part (b) of Fig. 10 is a diagram showing the time scale display information and the electronic medical record information updated in the electronic medical record display device.
[Fig. 11] Part (a) of Fig. 11 is a diagram showing the time scale display information and electronic medical record information displayed in the electronic medical record display device as the project management display device in the embodiment of the present invention, and Part (b) of Fig. 11 is a diagram showing the time scale display information and the electronic medical record information updated in the electronic medical record display device.
[Fig. 12] Part (a) of Fig. 12 is a diagram showing the time scale display information and electronic medical record information displayed in the electronic medical record display device as the project management display device in the embodiment of the present invention, and Part (b) of Fig. 12 is a diagram showing the time scale display information and the electronic medical record information updated in the electronic medical record display device.
[Fig. 13] Part (a) of Fig. 13 is a diagram showing a time machine display control screen utilized in the electronic medical record display device as the project management display device in the embodiment of the present invention, and Part (b) of Fig. 13 is a diagram showing time scale display information reflecting settings in the time machine display control screen.
[Fig. 14] Fig. 14 is a diagram showing time scale display information and electronic medical record information displayed in a uni-time display mode in the electronic medical record display device as the project management display device in the embodiment of the present invention.
[Fig. 15] Fig. 15 is a diagram showing the time scale display information and electronic medical record information in the uni-time display mode with all time scales displayed in a popup screen, in the electronic medical record display device as the project management display device in the embodiment of the present invention.
[Fig. 16] Fig. 16 is a diagram showing the time scale display information and electronic medical record information in the uni-time display mode with all time scales displayed in a popup screen, in the electronic medical record display device as the project management display device in the embodiment of the present invention.
[Fig. 17] Fig. 17 is a diagram showing the time scale display Information and electronic medical record information in the uni-time display mode with all time scales displayed in a popup screen, in the electronic medical record display device as the project management display device in the embodiment of the present invention.
[Fig. 18] Fig. 18 is a diagram showing time scale display information in which a period corresponding to a display period of electronic medical record information is highlighted in each time scale, in the electronic medical record display device as the project management display device in the embodiment of the present invention.
[Fig. 19] Fig. 19 is a diagram showing time scale display information with a subscale displayed therein, in the electronic medical record display device as the project management display device in the embodiment of the present invention.
[Fig. 20] Fig. 20 is a diagram showing time scale display information displayed by an electronic medical record display device as a project management display device in a. different embodiment of the present invention.
[Fig. 21] Fig. 21 is a diagram showing time scale display information displayed by the electronic medical record display device as the project management display device in the different embodiment of the present invention.
[Fig. 22] Fig. 22 is a diagram showing time scale display information displayed by the electronic medical record display device as the project management display device in the different embodiment of the present invention.
[Fig. 23] Fig. 23 is a diagram showing time scale display information displayed by the electronic medical record display device as the project management display device in the different embodiment of the present invention.
[Fig. 24] Fig. 24 is a diagram showing time scale display information displayed by the electronic medical record display device as the project management display device in the different embodiment of the present invention.

### MODES FOR CARRYING OUT THE INVENTION

An electronic medical record display device for displaying a treatment plan in an electronic medical record used in a medical field will be described as one embodiment of a project information display device of the present invention.

In the electronic medical record display device of this embodiment, medical information stored in an electronic medical record is displayed in accordance with each time scale indicating a time line in its corresponding time unit.

### <Configuration of Electronic Medical Record Display Device as One Embodiment>

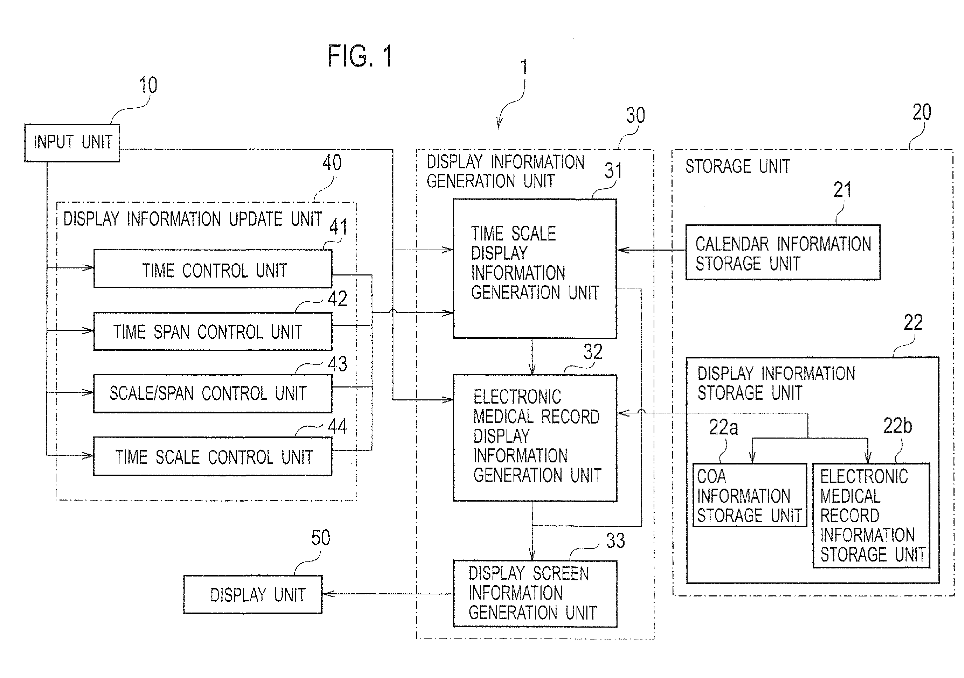

The configuration of an electronic medical record display device 1 in this embodiment will be described by referring to Fig. 1.

The electronic medical record display device 1 in this embodiment includes an input unit 10, a storage unit 20, a display information generation unit 30, a display information update unit 40, and a display unit 50.

The input unit 10 is manipulated by a medical doctor as a user and formed of a mouse, a stylus pen, a keyboard, and the like.

The storage unit 20 includes a calendar information storage unit 21 and a display information storage unit 22.

The calendar information storage unit 21 stores calendar information containing date information for displaying the time scale in decades, years, months, days, hours, minutes, or seconds.

The display information storage unit 22 includes a COA information storage unit 22a for storing a set of detailed information about various medical actions which is inputted in advance, and an electronic medical record information storage unit 22b for recording electronic medical record information in time units of decade, year, month, day, hour, minute, and second. The electronic medical record information is formed of: medical action information selected from the set of medical action information stored in the COA information storage unit 22a; and execution time information on a time at which the selected medical action is executed.

Description is now given of the set of medical action information stored in the COA information storage unit 22a.

In this embodiment, various medical actions to be displayed are defined and managed as "acts" individually.

Moreover, each of these acts contains information representing the core of the action (Core of Act; hereinafter, "COA")- In this COA, there are stored a relationship with different actions each defined based on a time line, and the like.

"Observation," "formulation," "injection," "feed," and the like are some types of acts. For each of these acts, there is information indicating a time based relationship with a different medical action. This "information indicating a time-based relationship with a different medical action" refers to information indicating, for example, that "observation" is required after the elapse of a predetermined period of time after "infection," or the like.

Information related to no medical action such for example as "symptom" is also a COA created based on an "observation" act. This information (symptom) is stored as a COA of "observation." In other words, every medical information is stored as a COA of some medical action (act).

Based on the above concept, in the electronic medical record display device of this embodiment, COAs each containing information based on a time line are inputted in and displayed with an electronic medical record which is a data structure already given the time line, thereby assisting the provision of highly precise medical information.

### - Configuration of COA -

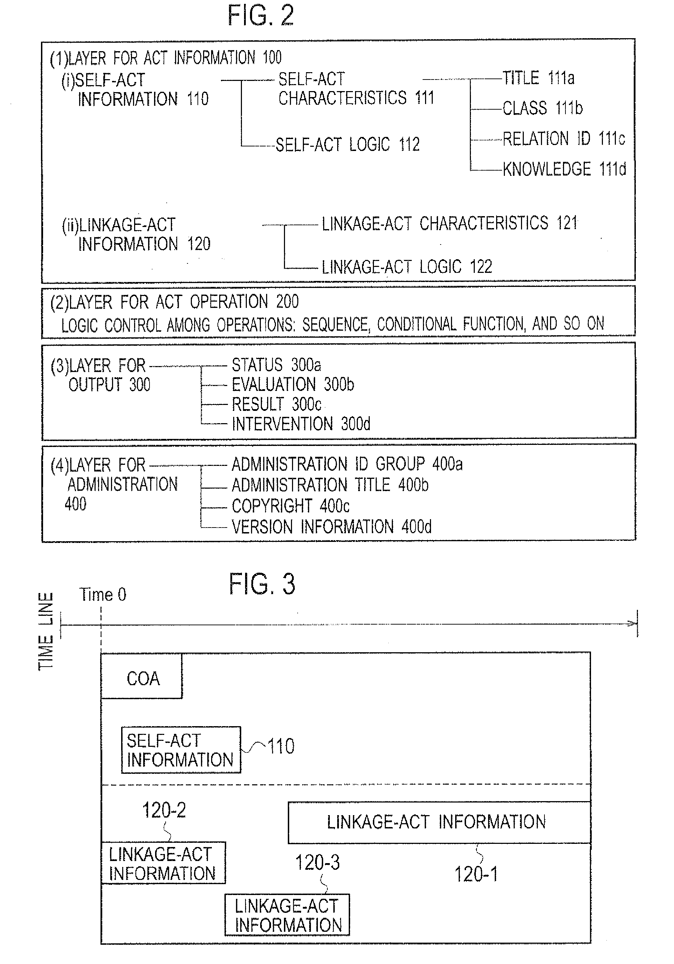

A set of information constituting each COA utilized in the present invention will be described by referring to Fig. 2.

Each COMA includes (1) a layer for act information 100, (2) a layer for act operation 200, (3) a layer for output 300, and (4) a layer for administration 400.

### (1) Layer for Act Information 100

The layer for act information 100 includes (i) self-act information 110 as self-action information being information on an act itself, and (ii) linkage-act information 120 as linkage information being information on linkage to a different COA.

### (i) Self-act information 110

The self-act information 110 further includes self-act characteristics 111 as information on characteristics of the action itself, and a self-act logic 112 as information on logic of the action itself. In the self-act characteristics 111, there are stored: a title 111a of the act itself (formal title, different title, temporary title, etc.); a class 111b (primary class, secondary class, tertiary class, etc.); a relation ID 111c (identification ID corresponding to the title 111a. and used for linkage to a different COA); and knowledge 111d (amount, rate, unit, scientific knowledge, know-how, etc. regarding the act itself).

In one example of the class 111b, the primary class includes "motional act" and "logical act," and the secondary class under the "motional act" includes "observational act" and "interventional act." Moreover, the tertiary class under the "observational act" includes "medical history," "medical condition," "finding," and the like. The tertiary class under the "interventional act" includes "laboratory test," "imaging test," "physiological test," "formulation," "injection," "treatment," "hospitalization/release," "freed," and the like. In addition, the tertiary class under the "logical act" includes "data generation by combination of test results," "prescription; issuing slip," and the like.

In the self-act logic 112, there are stored information on: a method of calculating an amount required when the self act is executed; a method of valuating the appropriate dose range and the medical condition; a method of converting and displaying a unit; and the like.

### (ii) Linkage act information 120

The linkage-act information 120 includes linkage-act characteristics 121 and linkage-act logic 122. In the linkage act characteristics 121, there is stored, on the basis of time-line information, information on a content, an applicable time, an applicable range, and the like of each dependent/exclusive action which is applied to a linkage of the COA with a different COA. In the linkage-act logic 122, there are stored a conditional function, a logic flow, and the like which use logical disjunction, logical conjunction, and the like and are used when the COA is linked to the different COA on the basis of the information of the linkage-act characteristics 121.

### (2) Layer for Act Operation 200

In the layer for act operation 200, there is stored logic control information on logic control among operations performed when processes are executed based on the self-act logic 112 and the linkage-act logic 122. The information contains an execution sequence, a conditional function, and the like, for example.

### (3) Layer for Output 300

In the layer for output 300, there are stored information such as a status 300a (studying, planning, execution determined, preparing, executing, result, etc.), evaluation 300b (success, failure, etc.), a result 300c (observation result, execution result, visualization, issuing slip/prescription, etc.), and intervention 300d for a different COA, all regarding the motion of the COA itself.

### (4) Layer for Administration 400

In the layer for administration 400, there are stored: an administration ID group 400a for managing each COA; an administration title 400b; a copyright 400c defining each COA as a publication; and version information 400d such as a writing date and an update date.

The applicable range of information of each COA on a time line is shown in Fig. 3. Each COA is allowed to include multiple linkage act information pieces 120. The COA in Fig. 3 includes three linkage acts corresponding to linkage-act information pieces 120-1 to 120-3.

In Fig. 3, the linkage-act information 120-1 is information which is applied after the elapse of a predetermined period of time after the execution of a medical action based on the self-act information 110. Moreover, the linkage act information 120-2 is information which is applied simultaneously with the execution of the medical action based on the self-act information 110. Furthermore, the linkage act information 120-3 is information which is applied from the time of the termination of the medical action based on the self-act information 110.

Referring back to Fig. 1, the display information generation unit 30 includes a time scale display information generation unit 31, an electronic medical record display information generation unit 32, and a display screen information generation unit 33.

The time scale display information generation unit 31 generates time scale display information on the basis of information acquired from the input unit 10 or the display information update unit 40. This time scale display information includes: a time scale display area to display time scales in multiple respective time units generated based on the calendar information of the calendar information storage unit 21; time span controller areas as display areas to perform an operation to lengthen or shorten the time spans of a time scale in the shortest time unit among the multiple time scales; scale/span controller areas as display areas to perform an operation to lengthen or shorten the time spans of the time scale in the shortest time unit among the multiple time scales and to shift the time units of the multiple time scales to their respective longer or shorter time units; and time scale control buttons as display areas to perform an operation to change the time units of the multiple time scales. In this embodiment, the time scale display information is generated such that time scales in three different time units may be displayed in the time scale display area.

Meanwhile, the time spans displayed in the time units in a default setting when the time scales are generated can be set to usable spans in advance if needed.

When the time scale display information is generated by the time scale display information generation unit 31, the electronic medical record display information generation unit 32 acquires, from the electronic medical record information storage unit 22b of the display information storage unit 22 in the storage unit 20, electronic medical record information corresponding to the time scale in the shortest time unit in the time scale display information and containing execution time information in the period displayed in this time scale, and generates electronic medical record display information. This electronic medical record display information is displayed corresponding to the time spans of the time scale in the shortest time unit. Moreover, when the time scale display information is updated by the later-described display information update unit 40, the electronic medical record display information generation unit 32 updates the electronic medical record information such that it may be displayed corresponding to the time spans of the updated time scale in the shortest time unit.

The display screen information generation unit 33 generates display screen information from the time scale display information generated by the time scale display information generation unit 31 and the electronic medical record display information generated by the electronic medical record display information generation unit 32 on the basis of the generated time scale display information.

The display information update unit 40 includes a time control unit 41, a time span control unit 42, a scale/span control unit 43, and a time scale control unit 44.

Upon detecting an operation to move horizontally the display periods in the time scales generated and displayed by the time scale display information generation unit 31 to periods before or after the display periods, the time control unit 41 updates the time scale display information such that the display periods in the time scales may be changed based on the operation.

Upon detecting an operation to lengthen or shorten the time spans of the time scale in the shortest time unit in one of the time span controller area generated and displayed by the time scale display information generation unit 31, the time span control unit 42 updates the time scale display information such that the time spans of the time scale in the shortest time unit in the time scale display area may be lengthened or shortened based on the operation.

Upon detecting an operation to lengthen or shorten the time spans of the time scale in the shortest time unit in one of the scale/span controller areas generated and displayed by the time scale display information generation unit 31, the scale span control unit 43 updates the time scale display information such that the time spans of the time scale in the shortest time unit in the time scale display area may be lengthened or shortened based on the operation. If the time spans are each lengthened to be longer than a predetermined time span, the time units of the multiple time scales are shifted to their respective shorter time units, whereas if the time spans are each shortened to be shorter than a predetermined time span, the time units of the multiple time scales are shifted to their respective longer time units.

Upon detecting an operation to change the time units of the displayed multiple time scales in one of the time scale control buttons generated and displayed by the time scale display information generation unit 31, the time scale control unit 44 updates the time scale display information such that a time unit may be selected from among preset multiple time units on the basis of the operation, changing the time units of the multiple time scales in the time scale display area.

The display unit 50 is one that displays the display screen information generated by the display screen information generation unit 33 of the display information generation unit 30, and is a display or the like.

### <Operations of Electronic Medical Record Display Device of One Embodiment>

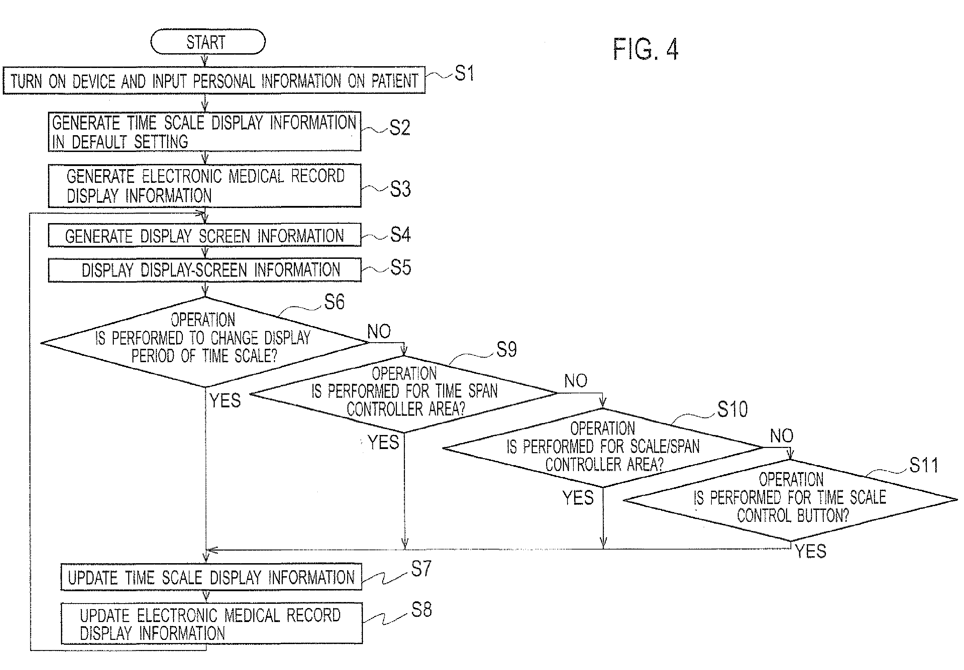

Operations of the electronic medical record display device 1 of the one embodiment will be described below in detail by referring to Figs. 4 to 19.

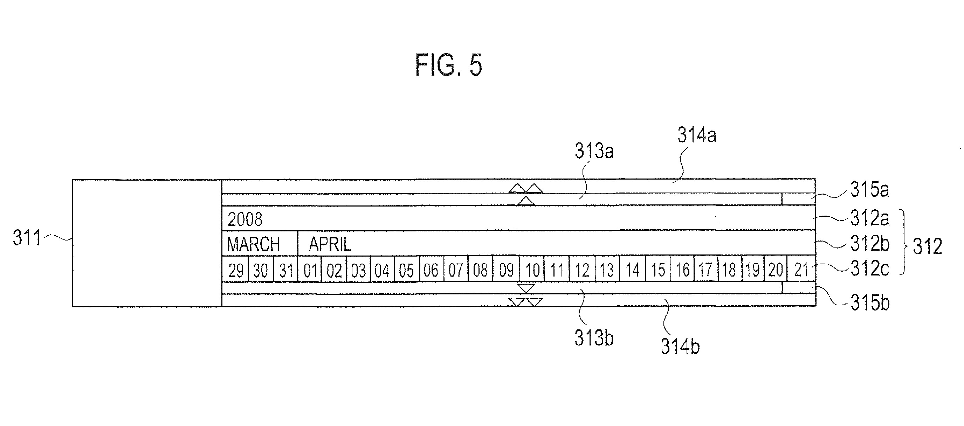

As shown in a flowchart in Fig. 4, a medical doctor first manipulates the input unit 10 to turn on the electronic medical record display device 1 and input personal information on a patient of interest (S1). Then, time scale display information 311 as shown in Fig. 5 is generated by the time scale display information generation unit 31 of the display information generation unit 30 on the basis of a default setting.

This time scale display information 311 is formed of a time scale display area 312, time span controller areas 313a and 313b, scale span controller areas 314a and 314b, and time scale control buttons 315a and 315b.

The time scale display area 312 acquires calendar information corresponding to a predetermined number of time units and displays corresponding time scales 312a to 312c.

The time span controller areas 313a and 313b are display areas to perform an operation to lengthen or shorten the time spans of the time scale 312c in the shortest time unit among the multiple displayed time scales, and are displayed at positions above and below the time scale display area 312.

The scale/span controller areas 314a and 314b are display areas to perform an operation to lengthen or shorten the time spans of the time scale 312c in the shortest time unit among the multiple time scales and to shift the time units of the multiple time scales to their respective longer or shorter time units, and are displayed at positions above and below the time scale display area 312.

The time scale control buttons 315a and 315b are display areas to perform an operation to change the time units of the multiple time scales 312a to 312c, and are displayed at positions above and below the time scale display area 312.

In this embodiment, in the default setting, the time scale display information 311 is generated with the time scales 312a to 312c in three different time units of year, month, and day indicating the continuous passages of time as shown in Fig. 5 (S2). In Fig. 5, the cell of the time scale 312c in days, which is the shortest time unit, displays 24 days from 29 Mar. 2008 to 21 Apr. 2008.

Thereafter, day-based electronic medical record information corresponding to a period from 29 Mar. 2008 to 21 Apr. 2008, which is the display period in the time scale 312c in the shortest time unit among all that are generated, is acquired by the electronic medical record display information generation unit 32 from the electronic medical record information storage unit 22b of the display information storage unit 22 in the storage unit 20. Once the electronic medical record information is acquired in the electronic medical record display information generation unit 32, electronic medical record display information 316 is generated such that it may be displayed corresponding to the day-based time scale 312c (S3).

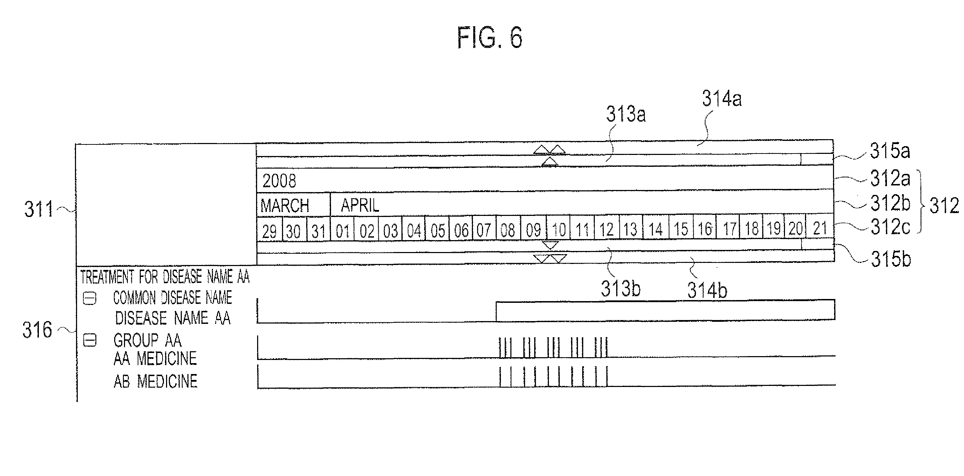

Subsequently, in the display screen information generation unit 33, display screen information is generated from the time scale display information 311 generated by the time scale display information generation unit 31 and the electronic medical record display information 316 acquired by the electronic medical record display information generation unit 32 (S4), and displayed on the display unit 50 as shown in Fig. 6 (S5).

Description will be now given of cases where the following operations are performed by manipulations on the input unit 10 by the medical doctor in the state as above where the display screen information formed of the time scale display information 311 and the electronic medical record display information 316 is displayed on the display unit 50. The operations are: (1) time warp operation for moving the display period; (2) time span control operation for lengthening or shortening the displayed time spans; (3) scale/span control operation for lengthening or shortening the display time spans and shifting the time units of the time scales to their respective longer or shorter time units; and (4) time scale control operation for changing the time units of the time scales.

### (1) Time Warp Operation

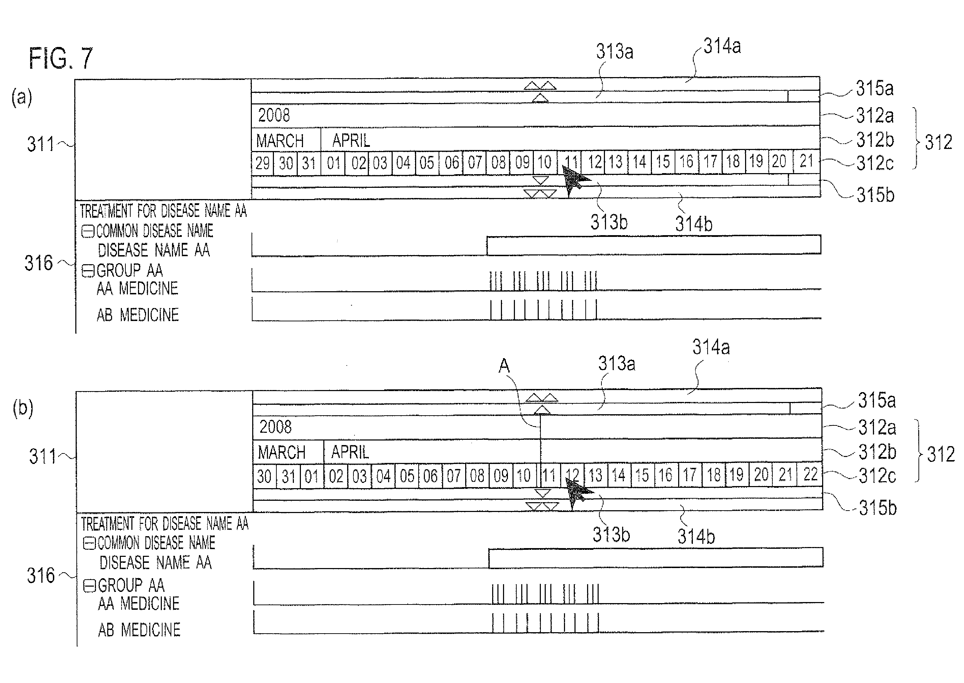

Assume that the medical doctor manipulates the input unit 10 by a click operation to specify, from the time scale display area 312 in the displayed time scale display information 311, the position of a date which he or she desires to display in the center of the screen, e.g., the position of 11 Apr. in the time scale 312c as shown in Part (a) of Fig. 7 ("YES" in S6). In this case, the time scale display information is updated by the time control unit 41 of the display information update unit 40 such that the period may be displayed with the position of 11 Apr. as the center (S7). This operation to specify the position desire to be displayed in the center can be performed on any of the time scales 312a to 312c. After the operation, all the displayed time scales 312a, 312b, and 312c are updated such that the specified position may be displayed as the center.

When the time scale display information 311 generated by the time scale display information generation unit 31 is updated as described, the electronic medical record display information 316 is updated by the electronic medical record display information generation unit 32 to information corresponding to the updated time scale display information 311, i.e., information of the given period with 11 Apr. set as the center (S8).

When the time scale display information 311 and the electronic medical record display information 316 are updated, the process returns to step S4, where display screen information is generated by the display screen information generation unit 33 on the basis of the information 311 and 316, and displayed on the display unit 50 as shown in Part (b) of Fig. 7 (S5, S6).

In this event, as shown in Part (b) of Fig. 7, an active line A being a line indicating the position of the date specified by the medical doctor may be set to be displayed on the displayed time scales. This active line A can function to indicate visually clearly the date specified by the medical doctor or function as an input position for a COA to be newly inputted.

### (2) Time Span Control Operation

Assume that the medical doctor manipulates the input unit 10 to perform an operation to lengthen or shorten the time spans of the time scale 312c in the shortest time unit at a desired position within the displayed time span controller area 313a or 312b (S9). In this case, the time scale display information 311 is updated by the time span control unit 42 such that the time spans of the time scale 812c may be lengthened or shortened about the date corresponding to the position at which the operation is performed (S7). This operation to lengthen or shorten the time spans is performed by a click operation with a mouse, a touch pen, or a touch pad or a scroll operation with a wheel mouse, for example.

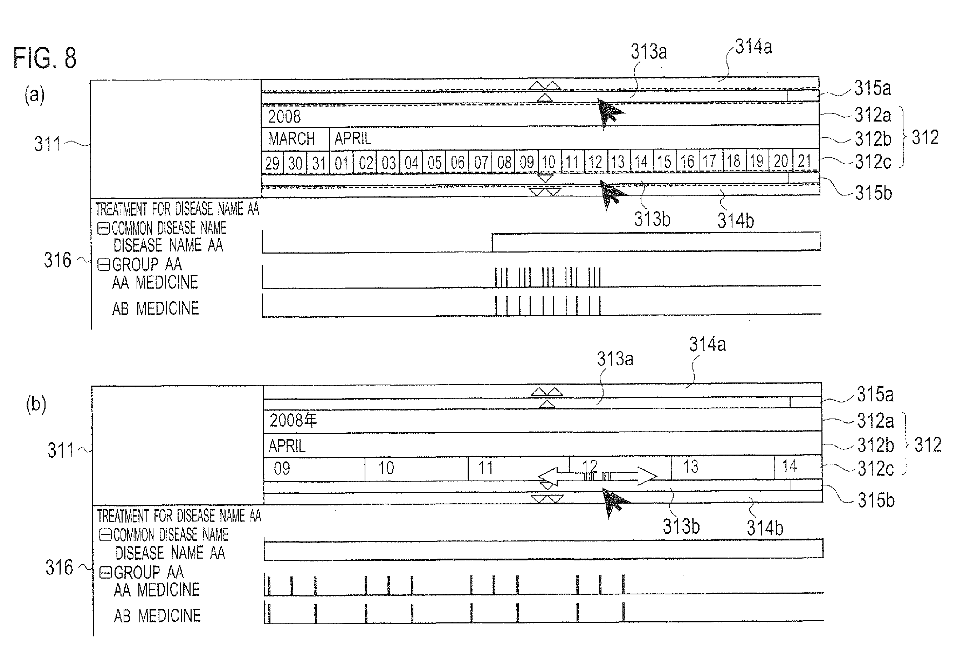

To be specific, as shown in Part (a) of Fig. 8, a click operation may be performed at a position corresponding to 12 Apr. 2008 in the time span controller area 313a displayed at the position above the time scale display area 312, or a scroll-up operation of a wheel mouse may be performed at a position corresponding to 12 Apr. 2008 in the time span controller area 313a or 313b displayed at the position above or below the time scale display area 312. Then, as shown in Part (b) of Fig. 8, the time scale display information is updated such that the time spans of the time scale 312c are lengthened about the position.

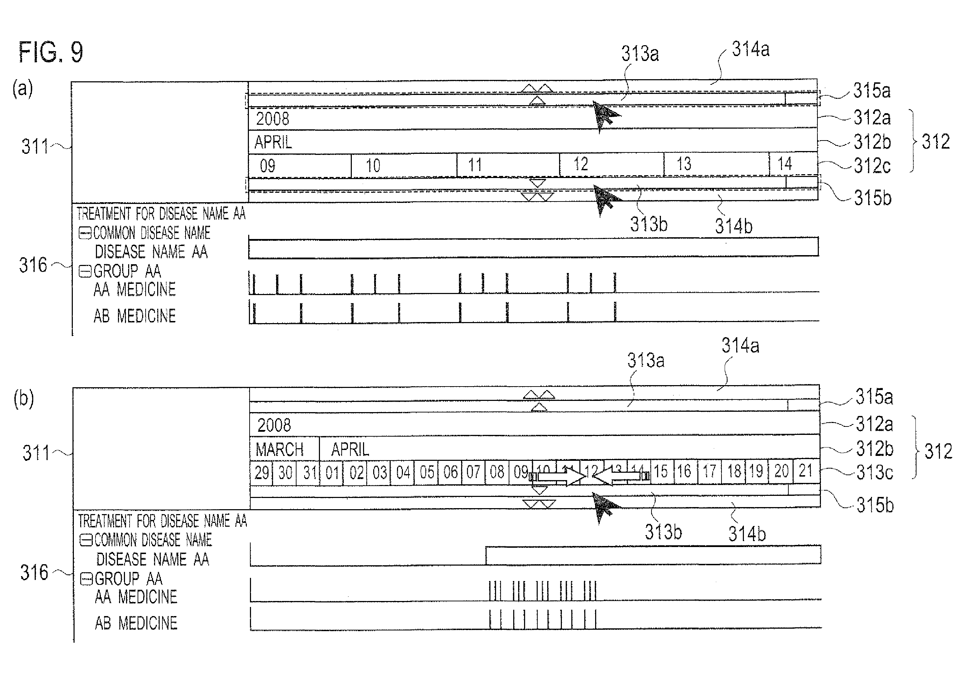

In addition, as shown in Part (a) of Fig. 9, a click operation may be performed at a position corresponding to 12 Apr. 2008 in the time span controller area 313b displayed at the position below the time scale display area 312, or a scroll-down operation of a wheel mouse may be performed at a position corresponding to 12 Apr. 2008 in the time span controller area 313a or 313b displayed at the position above or below the time scale display area 312. Then, as shown in Part (b) of Fig. 9, the time scale display information is updated such that the time spans of the time scale 312c are shortened about the position.

When the time scale display information 311 generated by the time scale display information generation unit 31 is updated as described, the electronic medical record display information 316 is also updated by the electronic medical record display information generation unit 32 to information of the given period corresponding to the updated time scale display information 311 (S8).

### (3) Scale/Span Control Operation

Assume that a medical doctor manipulates the input unit 10 to perform an operation to lengthen or shorten the time spans of the time scale 312c in the shortest time unit at a desired position in the displayed scale/span controller area 314a or 314b (S10). In this case, the time scale display information is updated by the scale/span control unit 43 of the display information update unit 40 such that the time spans of the time scale 312c may be lengthened or shortened about the date corresponding to the position at which the operation is performed (S7). In addition to this, if the time spans are each lengthened to be longer than a predetermined time span, the time units of the multiple time scales 312a to 312c are shifted to their respective shorter time units, whereas if the time spans are each shortened to be shorter than a predetermined time span, the time units of the multiple time scales 312a to 312c are shifted to their respective longer time units.

To be specific, in a state where the three time scales 312a to 312c are displayed respectively in years, months, and days in the time scale display area 312, the day-based time spans of the time scale 312c may be each lengthened to be longer than the preset span by performing the same operation as (2) time span control operation mentioned above within the scale/span controller area 314a or 314b. In this case, the time units in the time scale display area 312 are shifted to their respective shorter time units so that the three time scales may be displayed in months, days, and hours, for example, and the time scale display information is updated accordingly.

In addition, in a state where the three time scales 312a to 312c are displayed respectively in years, months, and days in the time scale display area 312, the day-based time spans of the time scale 312c may be each shortened to be shorter than the preset span by performing the same operation as (2) time span control operation mentioned above within the scale/span controller area 314a or 314b. In this case, the time units in the time scale display area 312 are shifted to their respective longer time units so that the three time scales may be displayed in centuries, years, and months, for example, and the time scale display information is updated accordingly.

When the time scale display information 311 generated by the time scale display information generation unit 31 is updated as described, the electronic medical record display information 316 is also updated by the electronic medical record display information generation unit 32 to information of the given period corresponding to the updated time scale display information 311 (S8).

In (2) time span control operation and (3) scale/span control operation described above, the description has been given of a case where the time scale display information is updated such that the time spans of the time scale 312c may be lengthened or shortened about the position at which the operation is performed. The update may also be performed such that the time spans of the time scale 312c may be lengthened or shortened in a state where the time scales are moved horizontally and the position at which the operation is performed is held at the center. In this case, the colors of operation icons in the time span controller areas 313a and 313b (triangle icons in Figs. 8, 9, etc.,) may be used to distinguish between the mode in which the time spans are lengthened or shortened about the position at which the operation is performed and the mode in which the time spans are lengthened or shortened in the state where the time scales are moved horizontally and the position at which the operation is performed is held at the center.

### (4) Time Scale Control Operation

Assume that the medical doctor manipulates the input unit 10 so that a click operation is performed on the time scale control button 315a or 315b of the displayed time scale display information 311 (S11). In this case, by the time scale control unit 44 of the display information update unit 40, the time units of the multiple time scales currently displayed in the time scale display area 312 are shifted to their respective longer or shorter time units, and the time scale display information is updated accordingly (S7).

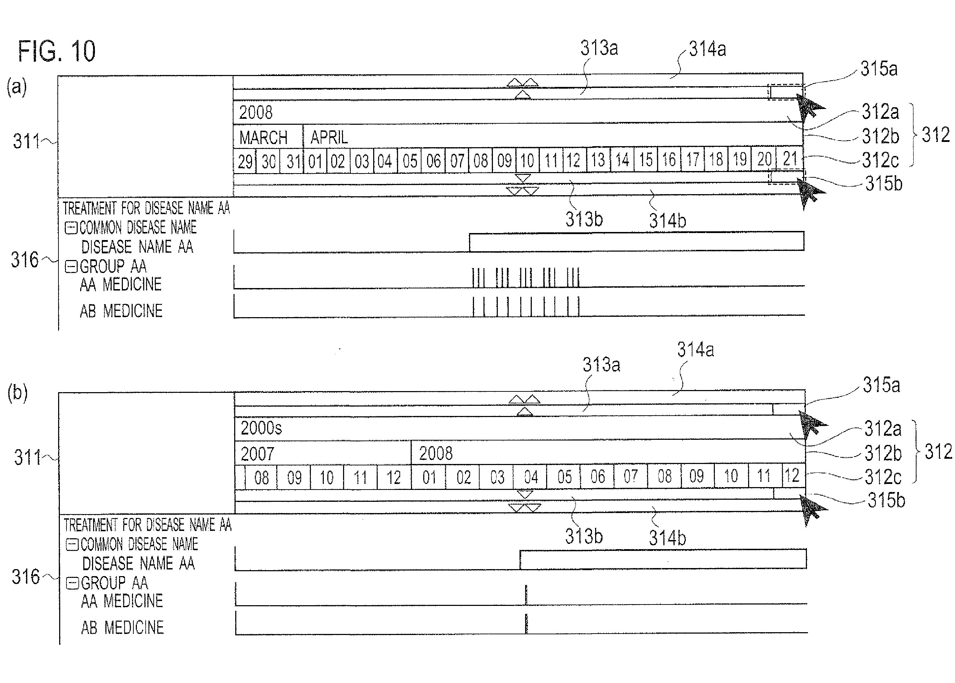

To be specific, as shown in Part (a) of Fig. 10, a click operation may be performed within the time scale control button 315a displayed at the position above the time scale display area 312, or a scroll-up operation of a wheel mouse may be performed within the time scale control button 315a or 315b displayed at the position above or below the time scale display area 312. In this case, the time units in the time scale display area 312 are shifted to their respective longer time units so that the three time scales are displayed respectively in centuries, years, and months, for example, and the time scale display information is updated as shown in Part (b) of Fig. 10.

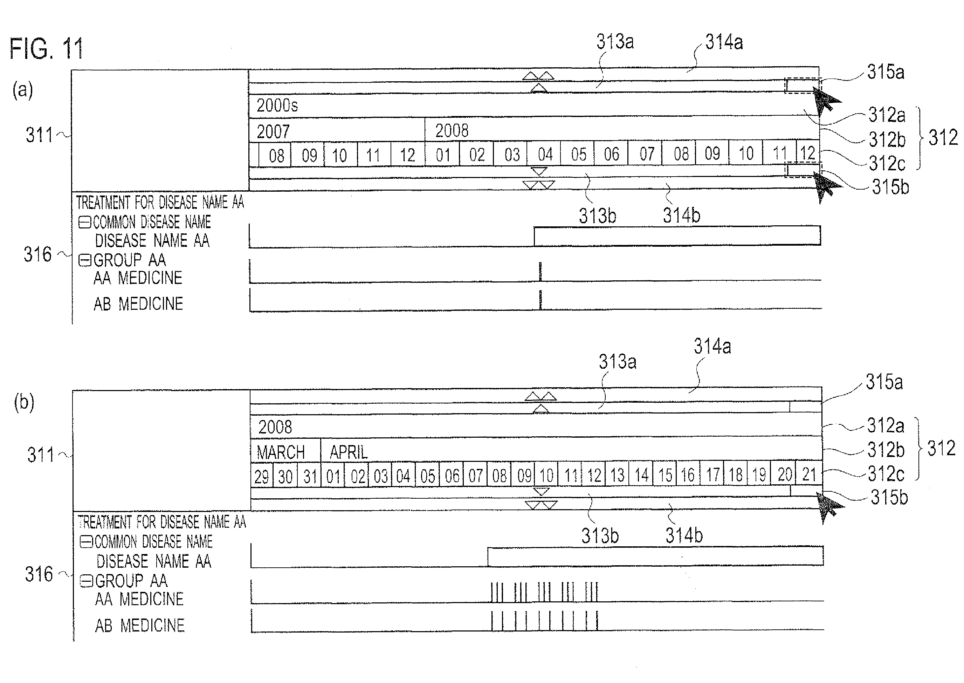

Moreover, as shown in Part (a) of Fig. 11, in the state where the three time scales 312a to 312c are displayed in centuries, years, and months in the time scale display area 312, a click operation may be performed within the time scale control button 315b displayed at the position below the time scale display area 312, or a scroll-down operation of a wheel mouse may be performed within the time scale control button 315a or 315b displayed at the position above or below the time scale display area 312. In this case, the time units in the time scale display area 312 are shifted to their respective shorter time units so that the three time scales are displayed in years, months, and days, for example, and the time scale display information is updated as shown in Part (b) of Fig. 11.

When the time scale display information 311 generated by the time scale display information generation unit 31 is updated as described, the electronic medical record display information 316 is also updated by the electronic medical record display information generation unit 32 to information of a given period corresponding to the updated time scale display information 311 (S8).

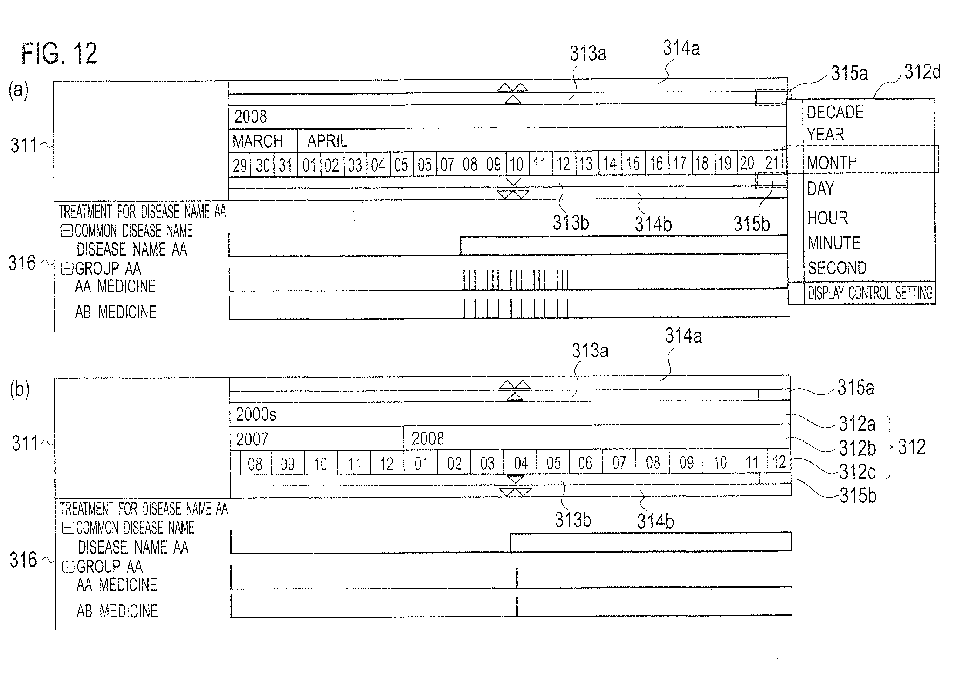

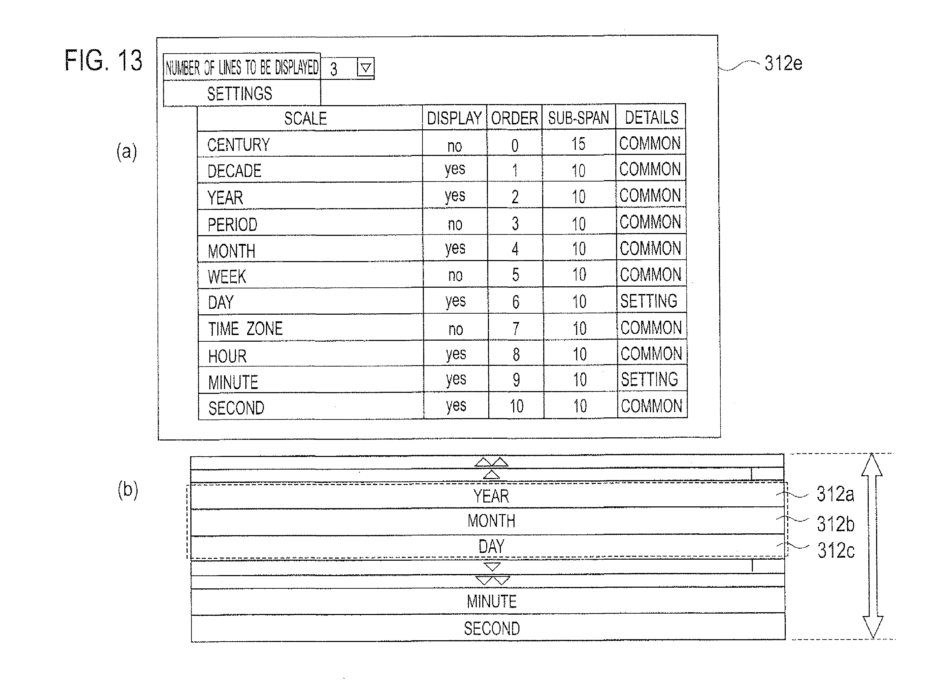

In this time scale control operation, the time units in the time scale display area 312 may be changed by selecting a desired time unit from a time unit selection menu 312d which is displayed as shown in Part (a) of Fig. 12 by right-clicking the time scale control button 315a or 315b or doing the like. The time units in the time scale display area 312 may be changed also by determining display information, such as desired time units and the number of lines to be displayed, in a time machine display control screen 312e which is displayed as shown in Part (a) of Fig. 13 by selecting "Display Control Settings" from the time unit selection menu 312d.

This time machine display control screen 312e is a screen for inputting setting information regarding the displaying of the time scales in the time scale display area 312. In one example of the display control screen shown in Fig. 13, "Settings" describe that, of multiple time scales in respective time units (scales) having "Display" set to "yes" and being listed in descending order of priority based on "Order," only N time scales are displayed, the N being a number set to "Number of Lines to be Displaced."

In Part (a) of Fig. 13, it is shown that, of the time scales in the time units having "display" set to "yes," i.e., "decade," "Year," "Month," "Day," "four," "Minute," and "Second," (time units within a bold arrow in Part (b) of Fig. 13), "3" time scales are selected and displayed as shown in Part (b) of Fig. 13, "3" being a number set to "Number of Lines to be Displayed."

### <Displaying Time Scale in Another Mode - 1>

In the embodiment described above, the description has been given of a case where the time scales 312a to 312c are displayed in three time units in the time scale display area 312 in the default setting. Instead, as another mode, only one time scale may be displayed as a base time scale. A function to display in only one time scale will be referred to as a "uni-time display" function.

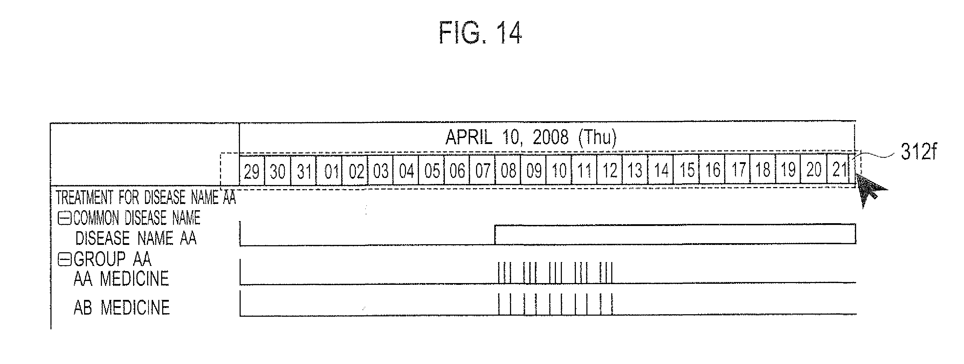

With the "uni-time display" function enabled, only one time scale is displayed in the time scale display area 312 as shown in Fig. 14, regardless of what is set to "Number of Lines to be Displayed."

Onmouse on an all time scale button 312f displayed in the vicinity of this one time scale displays, in a popup screen, all the time scales whose "Display" is set to "yes" in the time machine display control screen 312e.

This all time scale popup screen in the uni-time display mode is displayed in any one of the following three patterns.

### [All Time Scale Popup Screen: Pattern 1]

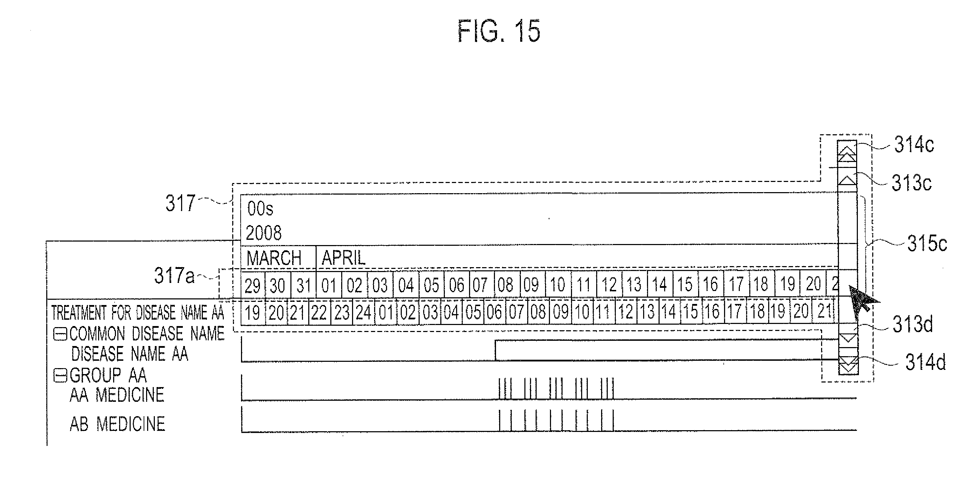

As shown in Fig. 15, the all time scale popup screen in Pattern 1 is configured to display all the times scales 317 whose "Display" is set to "yes" in the time machine display control screen 312e. Moreover, on the right ends of all the time scales 317, there are displayed: time span control buttons 313c and 313d for performing an operation to lengthen or shorten the time spans of the base time scale 317a; scale/span control buttons 314c and 314d for performing an operation to lengthen or shorten the time spans of the base time scale 317a and to shift the time unit of the base time scale to a longer or shorter time unit; and time scale control buttons 315c for changing the base time scale 317a.

Assume that all the time scales 317 and each of the aforementioned buttons are displayed and that a position which one desires to move to the center of the screen is specified from all the time scales 317 by a click operation. In this case, like (1) time warp operation described above, the time scale display information is updated to a state where the time scale is moved horizontally so that the period may be displayed with the specified position as the center.

Moreover, when a click operation is performed on the time span control button 313c or 313d, the time scale display information is updated such that the time spans may be lengthened or shortened about the center position of the base time scale 317a.

Moreover, when a click operation is performed on the scale/span control button 314c or 314d, the time scale display information is updated such that the time spans may be lengthened or shortened about the center position of the base time scale 317a. In addition, if the time spans are each lengthened to be longer than a predetermined time span, the time unit of the base time scale 317a is shifted to a shorter time unit, whereas if the time spans are each shortened to be shorter than a predetermined time span, the time unit of the base time scale 317a is shifted to a longer time unit.

Moreover, when a time scale control button 315c corresponding to one of the time scales is specified by a click operation, the time scale display information is updated by the time scale control unit 44 of the display information update unit 40 such that the base time scale may be changed to the time scale corresponding to the specified time scale control button 315c.

### [All Time Scale Popup Screen: Pattern 2]

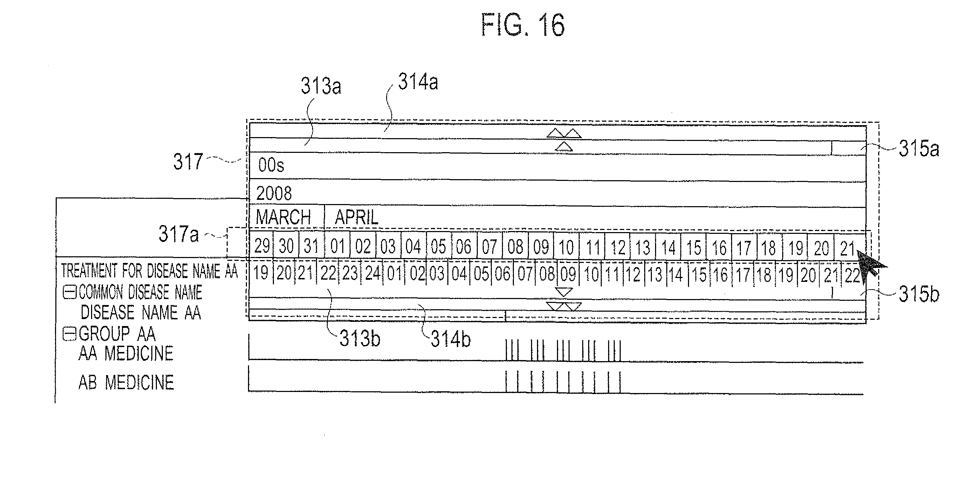

As shown in Fig. 16, the all time scale popup screen in Pattern 1 is configured to display all the times scales 317 whose "Display" is set to "yes" in the time machine display control screen 312e. Moreover, at positions above and below all the time scales 317, there are displayed: time span control buttons 313a and 313b for performing an operation to lengthen or shorten the time spans of the base time scale 317a; scale/span control buttons 314a and 314b for performing an operation to lengthen or shorten the time spans of the base time scale 317a and to shift the time unit of the base time scale to a longer or shorter time unit; and time scale control buttons 315a and 313b for shifting to the above and below time scales.

With all the time scales 317 and each of the aforementioned buttons, it is possible to perform the same operations as (1) time warp operation, (2) time span control operation, and (3) scale/span control operation described above.

### [All Time Scale Popup Screen: Pattern 3]

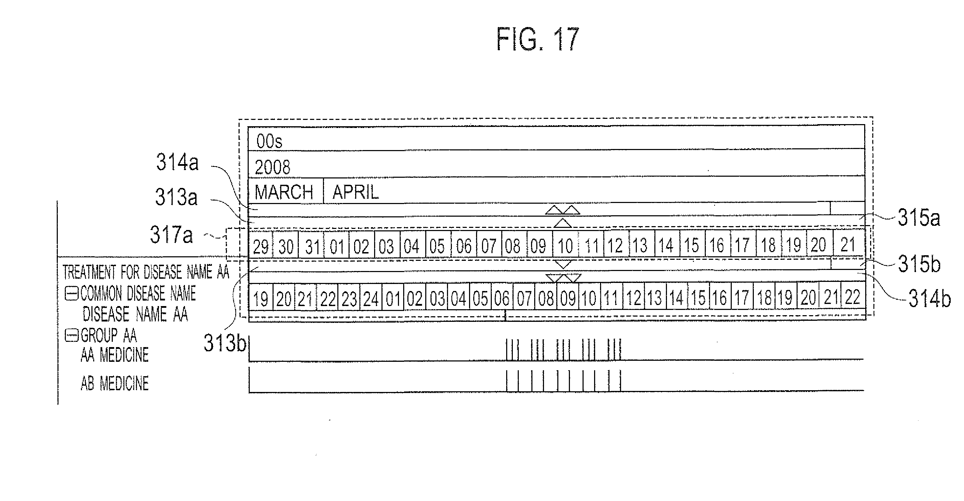

As shown in Fig. 17, the all time scale popup screen in Pattern 3 is configured to display the time span controller areas 313a and 313b, the scale/span controller areas 314a and 314b, and the time scale control buttons 315a and 315b at positions above and below the base time scale 317a when all the time scales 317 whose "Display" is set to "yes" in the time machine display control window 312e are displayed.

With all the time scales 317 and each of the aforementioned buttons, it is possible to perform the same operations as (1) time warp operation, (2) time span control operation, and (3) scale/span control operation described above.

When the time scale display information 311 is updated as described above, electronic medical record information corresponding to the display period in the time scale in the shortest time unit in the updated time scale display information 311 is acquired by the electronic medical record display information generation unit 32 from the electronic medical record information storage unit 22b, and the electronic medical record display information 316 is updated to be displayed corresponding to the time scale.

### <Displaying Time Scales in Another Mode - 2>

Meanwhile, the description has been given of a case where in step S2 in the above-described process, multiple time scales indicating the continuous passages of time are generated in the default setting. Instead, it is possible to generate time scales in each of which given periods selected by some operation of the medical doctor are arranged.

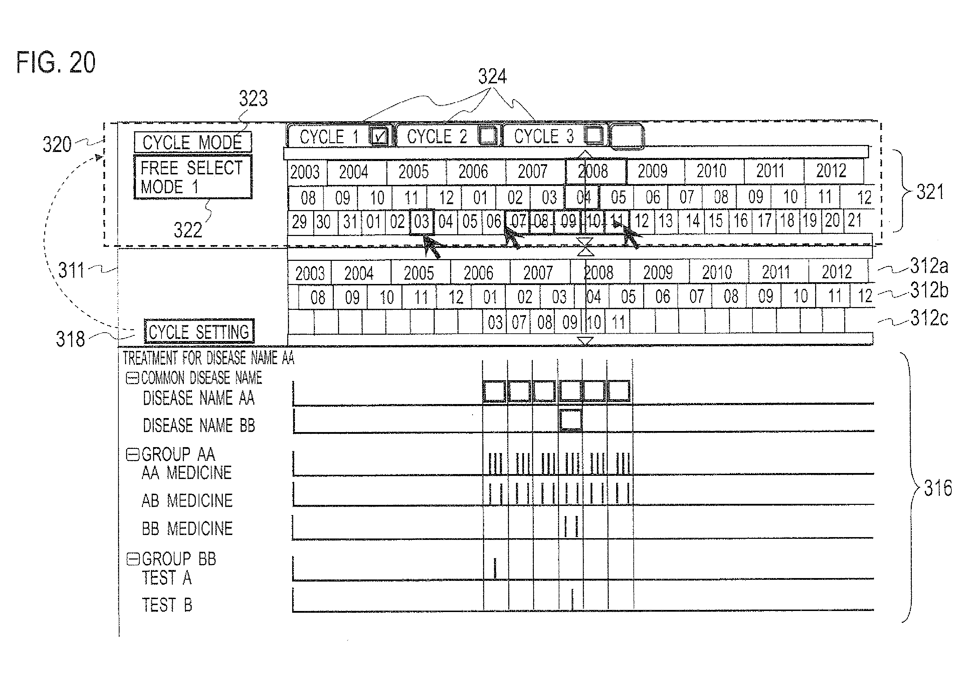

The given periods can be selected from time extra cycle setting information 320 as shown in Fig. 20. This time extra cycle setting information 320 is displayed by a click operation on a "Cycle Settling" button 318 displayed within the time scale display information 311.

This time extra cycle setting information 320 includes: time scales 321 used for selecting multiple time units and indicating the continuous passage of time; a "Free Select Mode" button 322 and a "Cycle Mode" button 323 for specifying a select mode, based on which given periods are to be selected from the time scales for selection 321; and cycle tabs 324 for storing or re-displaying information on the selected periods or cycles. In Fig. 20, three time scales in years, months, and days are displayed as the time scales for selection 321. The number of and the time units of the time scales for selection are changeable by changing the settings.

### [Free Select Mode]

Description will be now given of a process of selecting display periods in a case where the free select mode is specified as the selected mode in the displayed time extra cycle setting information 320.

In the free select mode, as the period desired to be displayed in each of the time scales 312a to 312c, multiple periods can be selected from the time scales for selection 321 in the time extra cycle setting information 320 by using independent period information.

For example, as shown in Fig. 20, if "3 Apr. 2008" and "7 Apr. 2008 to 11 Apr. 2008" are selected from the time scales for selection 321, the corresponding periods are highlighted. Further, the time scales 312a to 312c are updated based on the selected periods.

In the updated time scales 312a to 312c, periods corresponding to "3 Apr. 2008" and "7 Apr. 2008 to 11 Apr. 2008" selected from the time scales for selection 321 are displayed.

Here, in the time scale 312c in days, which is the shortest time unit among the time scales 312a to 312c, only the selected periods are arranged and displayed. The electronic medical record information 316 is updated accordingly so that only information corresponding to the selected periods may be displayed.

By specifying a cycle 1 tab of the cycle tabs 324 in the state where the display periods are selected as described, information of the display periods selected from the time extra cycle setting information 320 is stored in association with the cycle 1 tab.

### [Cycle Mode]

Description will be now given of a process of selecting display periods in a case where the cycle mode is specified as the selected mode in the displayed time extra cycle setting information 320.

In the free select mode, as the period desired to be displayed in each of the time scales 312a to 312c, multiple periods can be selected from the time scales for selection 321 in the time extra cycle setting information 320 by using period information relative to a reference time that comes periodically. This period information relative to a reference time that comes periodically refers to period information such as "every Thursday' and "every day from 9:00 to 19:00," for example.

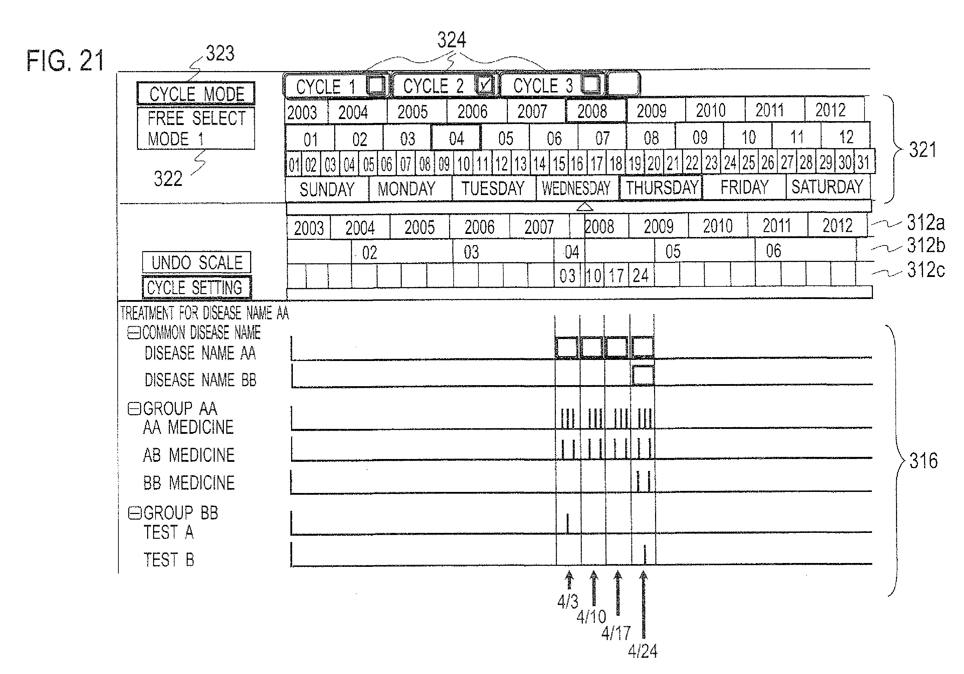

For example, as shown in Fig. 21, if "Thursdays Apr. 2008" are selected from the time scales for selection 321 in years, months, days and weeks, the corresponding periods are highlighted. Further, the time scales 312a to 312c are updated based on the selected periods.

In the updated time scales 312a to 312c, "3 Apr. 2008", "10 Apr. 2008", "17 Apr. 2008", and "24 Apr. 2008" selected from the time scales for selection 321 are displayed.

Here, in the time scale 312c in days, which is the shortest time unit among the time scales 312a to 312c, only the corresponding periods are arranged and displayed. The electronic medical record information 316 is updated accordingly so that only information corresponding to the selected periods may be displayed.

By specifying a cycle 2 tab of the cycle tabs 324 in the state where the display periods are selected as described, information of the display periods selected from the time extra cycle setting information 320 is stored in association with the cycle 2 tab.

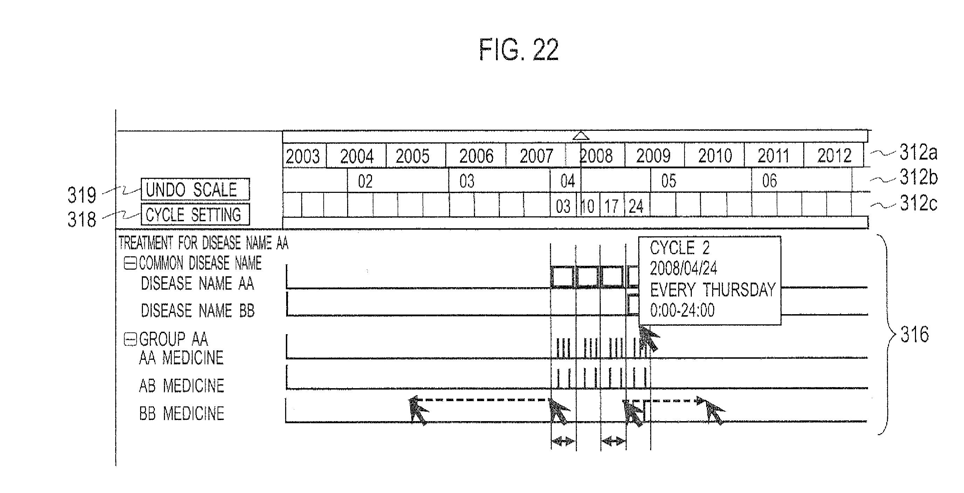

Moreover, the configuration may be such that placing a cursor over any blank portion of the electronic medical record information 316 on a screen where the time scales 312a to 312c and the electronic medical record information 316 are displayed as shown in Fig. 20 or 21, will display information on the display period at that position in a popup screen as shown in Fig. 22. In Fig. 22, "Cycle 2, 2008/04/24, every Thur., 0:00 - 24:00" is displayed in the popup screen as the information on the display period at the position.

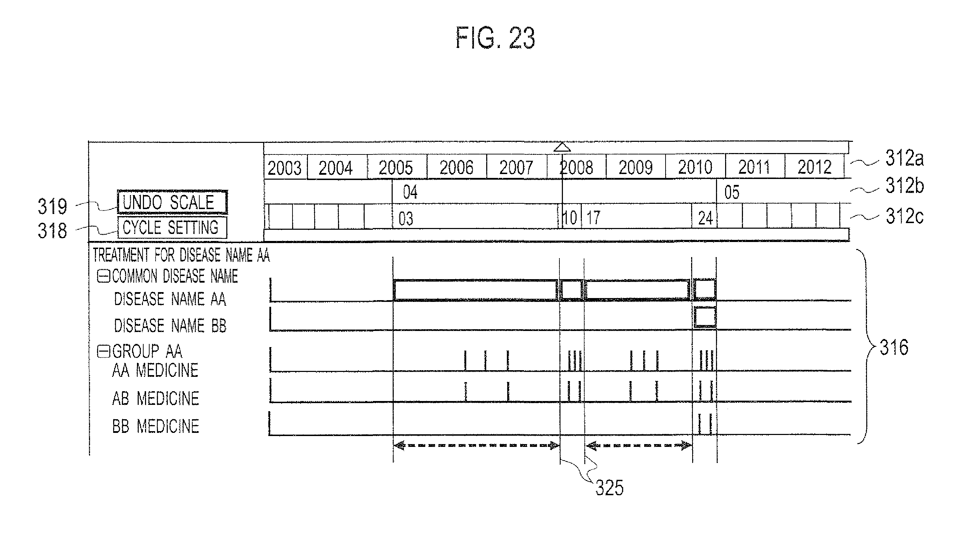

Moreover, the configuration may be such that performing a drag operation on a screen where the time scales 312a to 312c and the electronic medical record information 316 are displayed as shown in Fig. 20 or 21 will horizontally move partition lines 325 displayed corresponding to the time scale 312c in the electronic medical record information 316 and thereby displaying the display period at that position in a horizontally stretched fashion as shown in Fig. 23. In Fig. 23, the display periods of "3 Apr. 2008" and "17 Apr. 2008" are displayed stretched by distances by which the lines are moved.

The display periods stretched as shown in Fig. 23 return to their default setting states as shown in Fig. 22 by a click operation on an "Undo Scale" button 319.

Moreover, the configuration may be such that selecting multiple cycle tabs will display, on the same screen, the electronic medical record information 316 of all the display periods corresponding to the multiple cycle tabs.

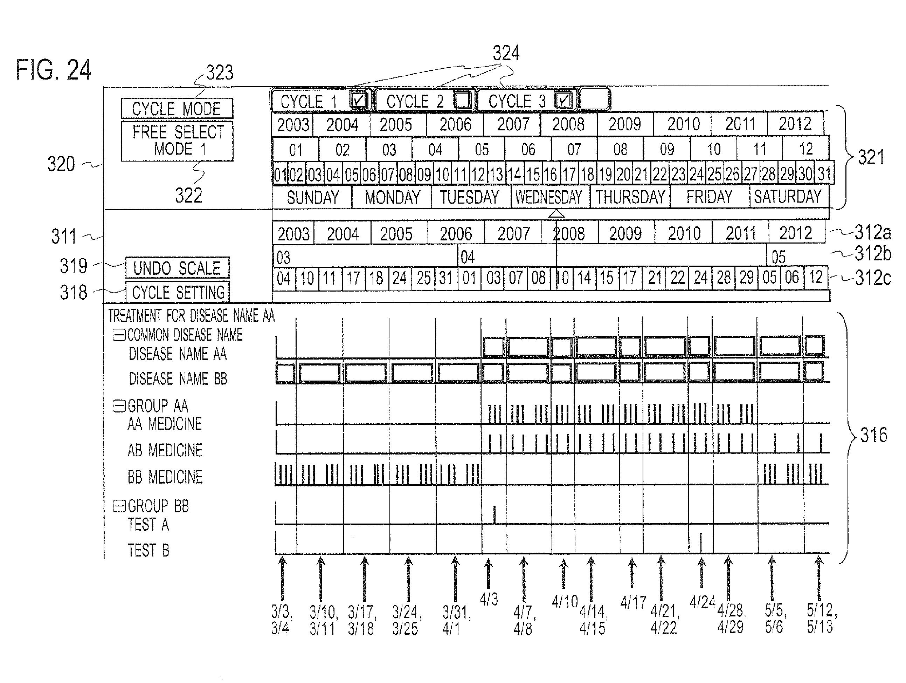

For example, in Fig. 24, the cycle 1 tab and a cycle 3 tab are selected. Accordingly, the time scales 312a to 312c are updated based on periods corresponding to both a display period "Thur. Apr. 2008" stored in association with the cycle 1 tab and a display period "Mon. and Tue. Mar. 2008 to May. 2008" stored in association with the cycle 3 tab.

Then, in the time scale 312c in days, which is the shortest time unit among the time scales 312a to 312c, all the corresponding periods are arranged and displayed. The electronic medical record information 316 is updated accordingly to display only the selected periods.

Since a set of electronic medical record information of only the given selected periods are arranged and displayed, it is possible to efficiently display a set of information of only desired periods.

Besides the method in which the given display periods are selected using a time scale of a given time unit as in the "free select mode" and "cycle mode" mentioned above, it is possible to employ a method in which the display periods are selected in days from the calendar information in which dates are displayed in weeks. Moreover, it is possible to employ a method in which the display periods are selected from a displayed time pattern formed of preset multiple time zones in a day.

Furthermore, when multiple time scales are displayed in the time scale display area 312 in the time scale display information 311 displayed as mentioned above, a period corresponding to a period currently displayed in the electronic medical record display information 316, i.e., a period displayed in the time scale with the shortest time unit, may be highlighted in the time scales in the respective time units.

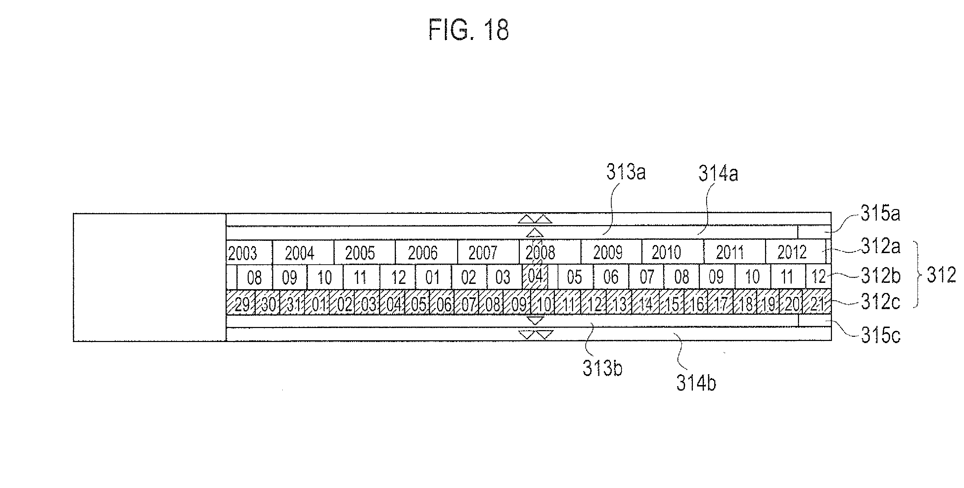

For example, when a period from 29 Mar. 2008 to 21 Apr. 2008 is displayed in the day-based time scale 312c which is a time scale in the shortest time unit at the moment, the following periods are highlighted as shown in Fig. 18: the whole display period in the time scale 312c; the period corresponding to 29 Mar. 2008 to 21 Apr. 2008 in the time scale 312b in months, which is a time unit higher by one level than the time scale 312c; and the period corresponding to 29 Mar. 2008 to 21 Apr. 2008 in the time scale 312a in years, which is a time unit further higher than the time scale 312c.

By highlighting the corresponding period in the time scales, the display period displayed currently in the electronic medical record display information 316 can be displayed in the time scales in the multiple time units visually recognizably.

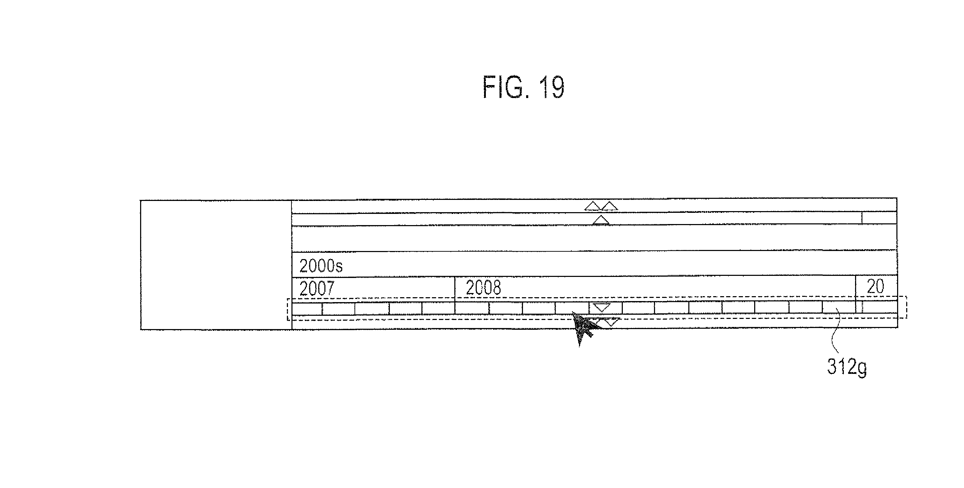

Assume that the medical doctor manipulates the input unit 10 to perform an operation to lengthen or shorten the time spans of the time scale 312c in the shortest time unit at a desired position within the displayed scale/span controller area 314a or 314b and that the time spans are each lengthened to be longer than a predetermined time span. In this case, a scale in a time unit lower by one level than the shortest time unit among those currently displayed may be displayed as a subscale as shown in Fig. 19, instead of shifting the time unit as described with reference to (3) scale/span control operation.

In Fig. 19, the operation is performed so that the time spans of the time scale in years, which is the shortest time unit among those currently displayed, are each lengthened to be longer than the predetermined time span; therefore, a scale in months, which is a time unit lower by one level than this year-based time scale, is displayed as a subscale 312g.

By displaying the subscale 312g as described, it is possible to roughly figure out a desired position in the shorter time unit without shifting the time unit of the time scale to the shorter time unit. Accordingly, it is possible to assist the operation required in performing the time warp operation or the time span control operation.

Meanwhile, in this embodiment, the description has been given of a case where in the time span control operation, the time scale display information is updated such that, of the time span controller areas 313a and 313b at two respective positions, a click operation on the time span controller area 313a displayed at the higher position will lengthen the time spans whereas a click operation on the time span controller area 313b displayed at the lower position will shorten the time spans. However, such functions may be reversed, and may be configured such that a click operation on the time span controller area 313a at the higher position will shorten the time spans whereas a click operation on the time span controller area 313b at the lower position will lengthen the time spans. The same applies to the scale/span control operation. The functions of the scale/span controller area 314a and the scale/span controller area 314b can be configured in reverse.

Moreover, in this embodiment, the description has been given of a case where three time units are selected in the time unit selection unit for the time scales displayed in the display screen. However, the number is not limited thereto, and can be changed to a different number such as four or two when needed.

Furthermore, in this embodiment, when time scales are displayed, they are listed in descending order of time unit from an upper portion of the screen. However, they may be displayed in reverse and listed in ascending order of time unit from the upper portion of the screen.

According to the electronic medical record information display device of this embodiment, electronic medical record information can be displayed which is generated corresponding to a time scale whose time spans are each changed by a simple operation to a time span a user desires.

In addition, a project information display program allowing a computer to function as the electronic medical record display device can be built by programming the functional configurations of the display information update unit, the display information generation unit, and the storage unit of the electronic medical record display device of the above-described embodiment and installing the program in the computer.

Moreover, in this embodiment, an electronic medical record display device has been described as an example of the project information display device. However, the project information display device is not limited thereto, and is applicable to any device that displays some other type of project information such for example as project information for managing manufacturing processes in a factory or schedule information in various fields, i.e., information that is based on a time line.

Furthermore, in this embodiment, the configuration is such that the storage unit is provided in the electronic medical record display device. However, the configuration is not limited thereto, and may be such that the storage unit is provided in a separate device such as a server connected via a network to the device including the display information manipulation unit and the display information generation unit.

By building such a configuration, it is possible to handle a case where the amount of information in the storage unit becomes extremely large.

Moreover, in this embodiment, the description has been given of a case where the time units of the time scales displayed on the display screen are selected from among decade, year, month, day, hour, minute, and second. However, the time units are not limited thereto. Time units such as week, thousand year, ten thousand year, hundred million year, millisecond, and microsecond can be selected in consideration of the contents to be displayed, as long as they are units representing times.

### EXPLANATION OF THE REFERENCE NUMERALS

- 1: electronic medical record display device
- 10: input unit
- 20: storage unit
- 21: calendar information storage unit
- 22: display information storage unit
- 22a: COA information storage unit
- 22b: electronic medical record information storage unit
- 30: display information generation unit
- 31: time scale display information generation unit
- 32: electronic medical record display information generation unit
- 33: display screen information generation unit
- 40: display information update unit
- 41: time control unit
- 42: time span control unit
- 43: scale/span control unit
- 44: time scale control unit
- 50: display unit
- 100: layer for act information
- 110: self-act information
- 111: self-act characteristics
- 112: self-act logic
- 120: linkage-act information
- 121: linkage-act characteristics
- 122: linkage-act logic
- 200: layer for act operation
- 300: layer for output
- 311: time scale display information
- 312: time scale display area
- 312a to 312c: time scale
- 312d: selection menu
- 312e: time machine display control screen
- 312f: all time scale button
- 312g: subscale
- 313a, 313b: time span controller area
- 313c: time span control button
- 314a, 314b: scale/span controller area
- 314c: scale/span control button
- 315a, 315b, 315c: time scale control button
- 316: electronic medical record display information
- 317: all time scales
- 317a: base time scale
- 400: layer for administration

## Claims

1. A project information display device **characterized by** comprising:
a storage unit configured to store calendar information, a plurality of action information pieces, and project information, the calendar information containing date information, the action information pieces being detailed information pieces on actions executed in a project, respectively, the project information including an action information piece selected from among the plurality of action information pieces and execution time information indicating a time at which the selected action information piece is executed;
a time scale display information generation unit configured to acquire, from the storage unit, the calendar information on a period specified as a display period and to generate time scale display information including a time scale display area for displaying a time scale, and a time span controller area being a display area for performing an operation to lengthen or shorten a time span of the time scale;
a display information update unit configured to update the time scale display information upon detecting the operation to lengthen or shorten the time span of the time scale, in the time span controller area of the time scale display information generated and displayed by the time scale display information generation unit, the time scale display information being updated such that the time span of the time scale in the time scale display area is lengthened or shortened based on the operation;
a project display information generation unit configured to acquire project information from the storage unit and to generate project display information when the time scale display information is generated by the time scale display information generation unit, and to update project display information when the time scale display information is updated by the display information update unit, the project information corresponding to the time scale in the time scale display information and containing the execution time information in the display period, the project display information allowing the project information to be displayed corresponding to the time span of the time scale, the project display information being updated such that the project information is displayed corresponding to the time span of the updated time scale;
a display information generation unit configured to generate display screen information including any one of the time scale display information generated by the time scale display information generation unit and the time scale display information updated by the display information update unit, and any one of the project display information generated by the project display information generation unit and the project display information updated by the project display information generation unit; and
a display unit configured to display the display screen information generated by the display information generation unit.

2. The project information display device according to claim 1, **characterized in that**
the time scale display information generated by the time scale display information generation unit further includes a scale/span controller area being a display area for performing an operation to lengthen or shorten the time span of the time scale and to shift a time unit of the time scale to a longer or shorter time unit, and
upon detecting the operation to lengthen or shorten the time span of the time scale in the scale/span controller area in the time scale display information generated and displayed by the time scale display information generation unit, the display information update unit updates the time scale display information such that the time span of the time scale in the time scale display area is lengthened or shortened on the basis of the operation and that the time unit of the time scale is shifted to the shorter time unit when the time span is lengthened to be longer than a predetermined time span whereas the time unit of the time scale is shifted to the longer time unit when the time span is shortened to be shorter than a predetermined time span.

3. The project information display device according to claim 1, **characterized in that**
the time span controller area in the time scale display information generated by the time scale display information generation unit is provided corresponding to time information of the time scale, and
upon detecting the operation to lengthen or shorten the time span of the time scale in the time span controller area, the time scale display information update unit updates the time scale display information in a way that while using, as a base point, date information in the time scale corresponding to a position within the time span controller area at which the operation is performed, a process is performed to lengthen or shorten the time span about any one of the position at which the operation is performed and a center position of the time scale.

4. The project information display device according to claim 2, **characterized in that**
the time span controller area in the time scale display information generated by the time scale display information generation unit is provided corresponding to time information of the time scale, and
upon detecting the operation to lengthen or shorten the time span of the time scale in the scale/span controller area, the time scale display information update unit updates the time scale display information in a way that while using, as a base point, date information in the time scale corresponding to a position within the scale/span controller area at which the operation is performed, a process is performed to lengthen or shorten the time span about any one of the position at which the operation is performed and a center position of the time scale.

5. The project information display device according to any one of claims 1 to 4, **characterized in that**
the time scale display information generated by the time scale display information generation unit further includes a time scale control button being a display area for performing an operation to change the time scale to a time scale in a time unit which is selected from among preset time units, and
upon detecting the operation to change the time unit of the time scale in the time scale control button, the display information update unit updates the time scale display information such that the time unit of the time scale in the time scale display area is changed based on the operation.

6. The project information display device according to any one of claims 1 to 5, **characterized in that**
the time scale displayed in the time scale display area is any one of a time scale indicating a continuous passage of time and a time scale in which given selected periods are arranged.

7. A project information display program causing a computer to execute:
a storage function configured to store calendar information, a plurality of action information pieces, and project information, the calendar information containing date information, the action information pieces being detailed information pieces on actions executed in a project, respectively, the project information being including an action information piece selected from among the plurality of action information pieces and execution time information indicating a time at which the selected action information piece is executed;
a time scale display information generation function configured to acquire, from information stored by the storage function, the calendar information on a period specified as a display period and to generate time scale display information including a time scale display area and a time span controller area, the time scale display area being where a time scale is displayed, the time span controller area being a display area for performing an operation to lengthen or shorten a time span of the time scale;
a display information update function configured to update the time scale display information upon detecting the operation to lengthen or shorten the time span of the time scale in the time span controller area of the time scale display information generated and displayed by the time scale display information generation function, the time scale display information being updated such that the time span of the time scale in the time scale display area is lengthened or shortened based on the operation;
a project display information generation function configured to acquire project information from information stored by the storage function and to generate project display information when the time scale display information is generated by the time scale display information generation function, and to update project display information when the time scale display information is updated by the display information update function, the project information corresponding to the time scale in the time scale display information and containing the execution time information in the display period, the project display information allowing the project information to be displayed corresponding to the time span of the time scale, the project display information being updated such that the project information is displayed corresponding to the time span of the updated time scale;
a display information generation function configured to generate display screen information including any one of the time scale display information generated by the time scale display information generation function and the time scale display information updated by the display information update function, and any one of the project display information generated by the project display information generation function and the project display information updated by the project display information generation function; and
a display function configured to display the display screen information generated by the display information generation function.

8. The project information display program according to claim 7, **characterized in that**
the time scale display information generated by the time scale display information generation function further includes a scale/span controller area being a display area for performing an operation to lengthen or shorten the time span of the time scale and to shift a time unit of the time scale to a longer or shorter time unit and
upon detecting the operation to lengthen or shorten the time span of the time scale in the scale/span controller area in the time scale display information generated and displayed by the time scale display information generation function, the display information update function updates the time scale display information such that the time span of the time scale in the time scale display area is lengthened or shortened on the basis of the operation and that the time unit of the time scale is shifted to the shorter time unit when the time span is lengthened to be longer than a predetermined time span whereas the time unit of the time scale is shifted to the longer time unit when the time span is shortened to be shorter than a predetermined time span.

9. The project information display program according to claim 7, **characterized in that**
the time span controller area in the time scale display information generated by the time scale display information generation function is provided corresponding to time information of the time scale, and
upon detecting the operation to lengthen or shorten the time span of the time scale in the time span controller area, the time scale display information update function updates the time scale display information by performing a process to lengthen or shorten the time span while using, as a base point, date information in the time scale corresponding to a position within the time span controller area at which the operation is performed, the time span being lengthened or shortened about any one of the position at which the operation is performed and a center position of the time scale.

10. The project information display program according to claim 8, **characterized in that**
the scale/span controller area in the time scale display information generated by the time scale display information generation function is provided corresponding to time information of the time scale, and
upon detecting the operation to lengthen or shorten the time span of the time scale in the scale/span controller area, the time scale display information update function updates the time scale display information by performing a process to lengthen or shorten the time span while using, as a base point, date information in the time scale corresponding to a position within the scale/span controller area at which the operation is performed, the time span being lengthened or shortened about any one of the position at which the operation is performed and a center position of the time scale.

11. The project information display program according to any one of claims 7 to 10, **characterized in that**
the time scale display information generated by the time scale display information generation function further includes a time scale control button being a display area for performing an operation to change the time scale to a time scale with a time unit which is selected from among preset time units, and
upon detecting the operation to change the time unit of the time scale in the time scale control button, the display information update function updates the time scale display information such that the time unit of the time scale in the time scale display area is changed based on the operation.

12. The project information display device according to any one of claims 7 to 11, **characterized in that**
the time scale displayed in the time scale display area is any one of a time scale indicating a continuous passage of time and a time scale in which given selected periods are arranged.

13. An electronic medical record information display device **characterized by** comprising:
a storage unit configured to store calendar information, a plurality of action information pieces, and project information, the calendar information containing date information, the action information pieces being detailed information pieces on actions executed in a project, respectively, the project information being including an action information piece selected from among the plurality of action information pieces and execution time information indicating a time at which the selected action information piece is executed;
a time scale display information generation unit configured to acquire, from the storage unit, the calendar information on a period specified as a display period and to generate time scale display information including a time scale display area and a time span controller area, the time scale display area being where a time scale is displayed, the time span controller area being a display area for performing an operation to lengthen or shorten a time span of the time scale;
a display information update unit configured to update the time scale display information upon detecting the operation to lengthen or shorten the time span of the time scale in the time span controller area of the time scale display information generated and displayed by the time scale display information generation unit, the time scale display information being updated such that the time span of the time scale in the time scale display area is lengthened or shortened based on the operation;
an electronic medical record display information generation unit configured to acquire electronic medical record information from the storage unit and to generate electronic medical record display information when the time scale display information is generated by the time scale display information generation unit, and to update electronic medical record display information when the time scale display information is updated by the display information update unit, the electronic medical record information corresponding to the time scale in the time scale display information and containing the execution time information in the display period, the electronic medical record display information allowing the electronic medical record information to be displayed corresponding to the time span of the time scale, the electronic medical record display information being updated such that the electronic medical record information is displayed correspondingly to the time span of the updated time scale;
a display information generation unit configured to generate display screen information including any one of the time scale display information generated by the time scale display information generation unit and the time scale display information updated by the display information update unit, and any one of the electronic medical record display information generated by the electronic medical record display information generation unit and the electronic medical record display information updated by the electronic medical record display information generation unit; and
a display unit configured to display the display screen information generated by the display information generation unit.
